## Europäisches Patentamt
## European Patent Office
### Office européen des brevets

(11) Veröffentlichungsnummer: **0 278 304**
**A1**

(12) # EUROPÄISCHE PATENTANMELDUNG

(21) Anmeldenummer: **88101038.3**

(22) Anmeldetag: **25.01.88**

(51) Int. Cl.⁴: **A61B 17/22** , A61B 8/00

(30) Priorität: **04.02.87 DE 3703334**

(43) Veröffentlichungstag der Anmeldung:
**17.08.88 Patentblatt 88/33**

(84) Benannte Vertragsstaaten:
**DE FR GB NL**

(71) Anmelder: **Siemens Aktiengesellschaft Berlin und München**
**Wittelsbacherplatz 2**
**D-8000 München 2(DE)**

(72) Erfinder: **Hassler, Dietrich**
**Flurweg 3**
**D-8525 Uttenreuth(DE)**
Erfinder: **Schmidt, Erhard**
**Heuwaagstrasse 20 A**
**D-8520 Erlangen(DE)**

(54) **Lithotripter mit integrierter Ortungseinrichtung.**

(57) Der Lithotripter zur Zertrümmerung eines Konkrements (19) im menschlichen Körper (17) umfaßt eine Stoßwellenimpulse aussendende Stoßwellenquelle (1) und eine Fokussierungseinrichtung (11). In Ausbreitungsrichtung der Stoßwellenimpulse gesehen vor der Fokussierungseinrichtung (11) ist unter festem Winkel (Alpha) ein Ultraschall-Spiegel (27) plaziert. Der Ultraschall-Spiegel (27) wird seitlich von einem Ultraschall-Wandler (21), der als Teil eines Sektorscanners ausgeführt ist, beschallt. Ein von der Stoßwellenquelle (1) ausgehender Stoßwellenimpuls wird durch den halbdurchlässigen Ultraschall-Spiegel (27) nur unwesentlich behindert, so daß die Therapie hiervon nahezu unbeeinflußt bleibt. Gleichzeitig wird das vom Ultraschall-Wandler (21) ausgesandte Ultraschall-Ortungssignal (31) von dem Ultraschall-Spiegel (27) in Richtung auf die Fokussierungseinrichtung (11) umgelenkt und von dort zur Beobachtung des Konkrements (19) und des umgebenden Gebietes verwendet. Als Vorteil ergibt sich die Möglichkeit einer kontinuierlichen Beobachtung der Lage des Konkrements (19) auch während der Zeit, in der die Stoßwellenimpulse im Konkrement (19) ihre Wirkung entfalten sowie eine Überwachungsmöglichkeit für den Zugangsweg des Stoßwellenimpulses zum Konkrement (19).

## Lithotripter mit integrierter Ortungseinrichtung

Die Erfindung betrifft einen Lithotripter zur Zertrümmerung eines Konkrements im menschlichen Körper mittels Stoßwellenimpulsen, die von einer Stoßwellenquelle erzeugt und von einer Fokussierungseinrichtung gebündelt sind.

Eine solche Vorrichtung ist beispielsweise aus der DE-OS 33 28 039 bekannt. Dort ist als Stoßwellenquelle ein Stoßwellenrohr eingesetzt, welches eine elektrische Spule, eine Isolierfolie und eine Kupfermembran umfaßt. Wird auf die Spule ein Stromimpuls gegeben, so werden in der vorgelagerten Membran Wirbelströme erzeugt, die die Membran von der Spule wegschlagen. In dem angrenzenden Übertragungsmedium, z.B. Wasser, bildet sich eine Stoßwelle aus. Diese wird durch eine akustische Linse fokussiert, deren Brennpunkt sich nach einem Einstellvorgang im Konkrement des Patienten befindet. Bei dem Konkrement handelt es sich beispielsweise um einen Nierenstein.

Die Ortung des Konkrements hat dabei eine hohe Bedeutung. Je größer die Zielgenauigkeit ist, desto größer ist der therapeutische Erfolg und desto kleiner die Belastung des Patienten. Es ist bekannt, die Ortung mit Röntgengeräten vorzunehmen. Allerdings kann dabei wegen der Strahlenbelastung nicht während der gesamten Stoßwellenbehandlung die Lage des Steins überprüft werden. Es werden jeweils nur von Zeit zu Zeit Röntgenbilder aufgenommen, um die Lage des Steins zu kontrollieren. Für eine kontinuierliche Überwachung der Steinlage ist man deswegen bereits dazu übergegangen, die Ortung des Konkrements mit Ultraschall vorzunehmen. So zum Beispiel ist aus der DE-PS 34 27 001 ein Ortungs-und Positionierverfahren bekannt, bei welchem mittels eines Ultraschall-Schwingers das Konkrement geortet wird, vorgegebene Markierungsmarken gesetzt werden und an schließend eine mechanische Korrelation der Position des Konkrements mit dem Brennpunkt des Stoßwellensystems vorgenommen wird.

Weiterhin ist es aus der DE-OS 31 19 295 bekannt, die Ortung des Konkrements mit der Stoßwellenquelle selbst vorzunehmen. Im dort geschilderten System ist die Stoßwellenquelle eine Anordnung einer Vielzahl piezoelektrischer Wandlerelemente. Dieses Verfahren kann allerdings nur bei Stoßwellenquellen, deren Stoßwellenimpuls mit piezoelektrischen Elementen hervorgerufen wird, angewendet werden.

Aufgabe der Erfindung ist es daher, einen Lithotripter der eingangs genannten Art so auszugestalten, daß eine fortlaufende Ultraschall-Ortung des Konkrements unabhängig von der Stoßwellenquelle auch während der Einwirkung des Stoßwellenimpulses möglich ist.

Diese Aufgabe ist erfindungsgemäß dadurch gelöst, daß auf der Zentralachse zwischen der Stoßwellenquelle und der Fokussierungseinrichtung ein akustisch halbdurchlässiger Spiegel angeordnet ist, und daß seitlich zur Zentralachse der Ultraschall-Wandler eines Ultraschall-Sektorscanners angeordnet ist, der zur Beschallung des Spiegels vorgesehen ist.

Der Spiegel ist bevorzugt eben ausgebildet. Wird dagegen eine geeignete Krümmung verwendet, so läßt sich dadurch die Scanform beeinflussen.

Beim Durchlauf eines Stoßwellenimpulses durch den halbdurchlässigen Spiegel wird der Stoßwellenimpuls nur unwesentlich gedämpft. Es gibt an beiden Grenzflächen (Vorder-und Rückseite) Reflexe. Der Spiegel sollte daher relativ dünn sein, da die Überlagerung der beiden Reflexe, zwei in Schallrichtung gegeneinander verschobene Bilder erzeugt, deren Verschiebung umso geringer ist, desto dünner der Spiegel gewählt wurde. Der Verlust an Energie durch Reflexion und Dämpfung am Spiegel kann im Sendefall des Stoßwellenimpulses und des Ortungsimpulses durch mehr Sendeenergie ausgeglichen werden.

Man kann durch die Wahl des Wellenwiderstands des Spiegels einstellen, wieviel Schallenergie durchgelassen und wieviel reflektiert wird. Die Dämpfung des Stoßwellenimpulses im Spiegel kann durch eine höhere Anfangsamplitude ausgeglichen werden. Ansonsten nimmt die Stoßwelle einen durch den Ultraschall-Spiegel ungehinderten Verlauf. Gleichzeitig kann über den seitlich angeordneten Sektorscanner das vom Ultraschall-Wandlerelement erzeugte Ultraschall-Ortungssignal vom Spiegel in Richtung auf die Fokussierungseinrichtung umgelenkt werden, um das zu untersuchende Gebiet zu beschallen. Die reflektierten Echos werden ebenfalls vom Spiegel umgelenkt und von dem Ultraschall-Wandler des Ultraschall-Sektorscanners empfangen. Auf diese Weise ist eine kontinuierliche Beobachtung, also auch während der Wirkung des Stoßwellenimpulses, möglich.

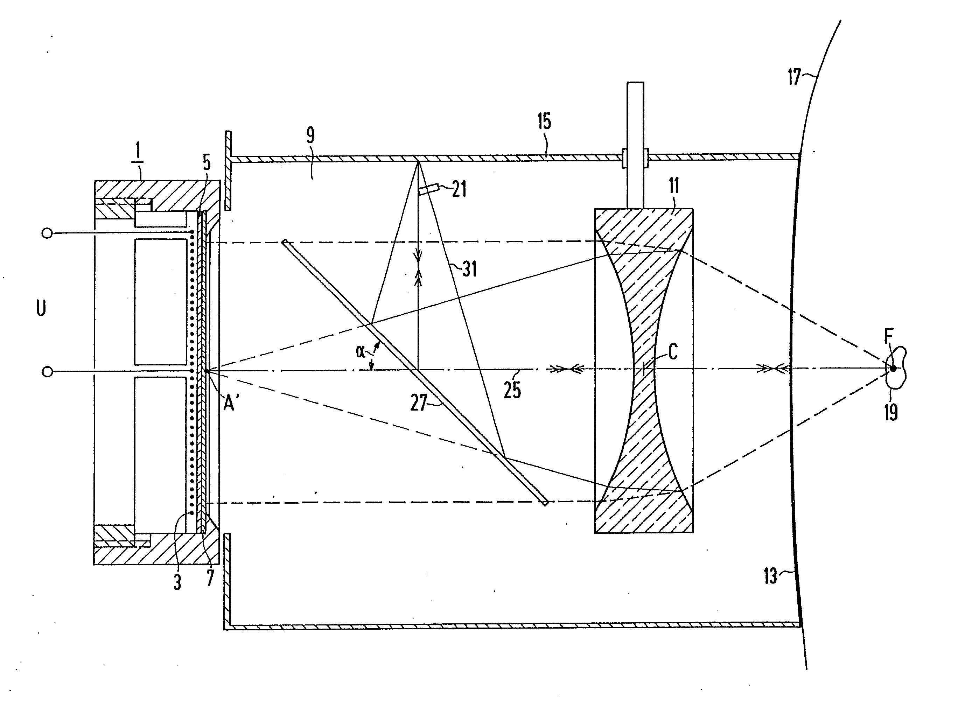

Weitere Vorteile und Ausgestaltungen der Erfindung ergeben sich aus der Beschreibung eines Ausführungsbeispiels anhand der Figur.

Die Figur zeigt einen Lithotripter mit integrierter Ortungseinrichtung.

In der Figur ist eine an sich bekannten Stoßwellenquelle 1 dargestellt. Die Stoßwellenquelle 1 umfaßt eine flache elektrische Spule 3, vor der, durch eine Isolierfolie 5 getrennt. eine Kupfermembran 7 angeordnet ist. Die Spule 3 kann mit einem Spannungsimpuls U beaufschlagt werden. An die Kupfermembran 7 schließt sich eine Wasservorlaufstrecke 9 an. In der Wasservorlaufstrecke 9 befindet sich als Fokussierungseinrichtung eine Linse 11. Die Wasservorlaufstrecke 9 ist stirnseitig von einer Ankoppelmembran 13 abgeschlossen. Ein zylindrisches Rohrteil 15, welches stirnseitig zum einen von der Stoßwellenquelle 1 und zum anderen von der Ankoppelmembran 13 abgeschlossen ist, bildet ein Gehäuse. Dieses ist mit der Koppelflüssigkeit Wasser gefüllt.

Die Ankoppelmembran 13 ist an die Hautoberfläche eines Patienten 17 angelegt. Die Stoßwellenquelle 1 ist dabei so positioniert, daß der zweite Fokus F der Linse 11 mit der Lage eines Konkrements 19, z.B. eines Nierensteins, zusammenfällt.

In Ausbreitungsrichtung gesehen vor der Linse 11 befindet sich ein halbdurchlässiger, ebener Ultraschall-Spiegel 27. Der Ultraschall-Spiegel 27 ist vorzugsweise um einen Winkel Alpha = 45° zur Zentralachse 25 der Fokussierungseinrichtung oder Linse 11 gekippt und auf etwa halbem Wege zwischen Stoßwellenquelle 1 und Linse 11 fest plaziert. Dem Ultraschall-Spiegel 27 liegt ein am Rohrteil 15 befestigter Ultraschall-Wandler 21 seitlich gegenüber, der Teil eines mechanischen Sektorscanners ist. Der Ultraschall-Wandler 21 des Sektorscanners ist schwenkbar um eine Drehachse, die im Punkt A senkrecht auf der Papierebene steht. Der Punkt A kann etwa auf der Wand des Gehäuses liegen. Die vom Ultraschall-Wandler 21 ausgehenden, nacheinander fächerförmig ausgesandten Ultraschall-Ortungssignale 31 werden vom Ultraschall-Spiegel 27 in Richtung auf die Linse 11 umgeleitet. Durch die Linse 11 werden sie in einen konvergierenden Sektorscan umgeformt. Das Bildgebersystem ist dem konvergierenden Sektorscan angepaßt und stellt nicht wie beim Sektorscan einen Divergent-Scan dar.

Bevorzugt ist der Ultraschall-Spiegel 27 zusammen mit dem Sektorscanner 21 um die Zentralachse 25 drehbar ausgeführt, so daß sich im Patienten 17 ein kegelstumpfförmiges Untersuchungsgebiet beobachten läßt.

Der Ultraschall-Spiegel 27 ist möglichst dünn gehalten, damit Mehrfachreflekionen im Spiegel 27 selbst möglichst wenig in Erscheinung treten. Die Dicke sollte kleiner als 0,5 mm sein. Als Material für den Ultraschall-Spiegel 27 kommt beispielsweise ein Kunststoff, wie Plexiglas, in Frage. Die Lage des Ultraschall-Spiegels 27 zwischen der Stoßwellenquelle 1 und der Fokussierungseinrichtung 11 ist vorzugsweise so gewählt, daß der scheinbare Ursprung A' der vom Ultraschall-Wandler 21 nacheinander sektorförmig ausgesandten Ultraschall-Ortungsstrahlen 31 auf der Abstrahlfläche der Stoßwellenquelle 1, also auf der Kupfermembran 7, liegt.

Der konvergierende Sektorscan auf Seiten des Patienten 17 wird durch geeignete Wahl des Abstands des Ultraschall-Spiegels 27 zwischen der Kupfermembran 7 der Stoßwellenquelle 1 und der Fokussierungseinrichtung oder Linse 11 erreicht.

Vorteil des dargestellten Lithotripters mit integrierter Ortungseinrichtung ist es, daß während der gesamten Behandlung das Konkrement 19 beobachtbar bleibt. Dies gilt auch für die Dauer des Stoßwellenimpulses selbst und für die Zeit seiner Einwirkung auf das Konkrement 19. Hinzu kommt, daß der Schallweg des Stoßwellenimpulses im ganzen Volumen ebenfalls beobachtet und geeignet ausgewählt werden kann.

Vorteilhafterweise ist noch eine (nicht gezeigte) Synchronisationseinheit vorgesehen, so daß ein Stoßwellenimpuls dann ausgelöst wird, wenn das vom Stein 19 herrührende Echo dieses Stoßwellenimpulses von Sektorscan-Wandler 21 empfangen werden kann. Zu einem solche Zeitpunkt "schaut" das Ultraschall-System gerade auf den Stein 19. Dies kann ein zusätzliches Zeit- und/oder Erfolgskriterium bei der Konkrement-Therapie sein.

## Ansprüche

1. Lithotripter zur Zertrümmerung eines Konkrements im menschlichen Körper mittels Stoßwellenimpulsen, die von einer Stoßwellenquelle erzeugt und von einer Fokussierungseinrichtung gebündelt sind, **dadurch gekennzeichnet,** daß auf der Zentralachse (25) zwischen der Stoßwellenquelle (1) und der Fokussierungseinrichtung (11) ein akustisch halbdurchlässiger Spiegel (27) angeordnet ist, und daß seitlich zur Zentralachse (25) der Ultraschall-Wandler (21) eines Ultraschall-Sektorscanners angeordnet ist, der zur Beschallung des Spiegels (27) vorgesehen ist.

2. Lithotripter nach Anspruch 1, **dadurch gekennzeichnet,** daß der Spiegel (27) zusammen mit dem Ultraschall-Wandler (21) um die Zentralachse (25) drehbar ist.

3. Lithotripter nach Anspruch 1 oder 2, **dadurch gekennzeichnet,** daß der Spiegel (27) unter einem Winkel (Alpha) von 45° zur Zentralachse (25) gekippt ist.

4. Lithotripter nach einem der Ansprüche 1 bis 3, **dadurch gekennzeichnet,** daß der Spiegel (27) weniger als 0,5 mm dick ist.

5. Lithotripter nach einem der Ansprüche 1 bis 4, **dadurch gekennzeichnet,** daß der Spiegel (27) aus einem Kunststoff besteht.

6. Lithotripter nach einem der Ansprüche 1 bis 4, **dadurch gekennzeichnet,** daß der scheinbare Ursprung (A') des vom Ultraschall-Wandler (21) ausgesandten Ultraschall-Ortungstrahls (31) auf der Abstrahlfläche (7) der Stoßwellenquelle (1) liegt.

7. Lithotripter nach einem der Ansprüche 1 bis 6, **dadurch gekennzeichnet,** daß der Abstand zwischen der Abstrahlfläche (7) der Stoßwellenquelle (1) und der Fokussierungseinrichtung (11) so gewählt ist, daß die vom Ultraschall-Wandler (21) bei sektorförmiger Bewegung vor dem Spiegel (27) ausgesandten Abtaststrahlen (31) im Gebiet hinter der Fokussierungseinrichtung (11) konvergieren.

8. Lithotripter nach einem der Ansprüche 1 bis 7, **dadurchgekennzeichnet,** daß der Ultraschall-Wandler (21) ein mechanisch beweglicher Sektor-Abtastkopf ist.

9. Lithotripter nach einem der Ansprüche 1 bis 8, **dadurch gekennzeichnet,** daß der Spiegel (27) eben ist.

87 P 3022

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 716 826 (P. GREEN) <br> * Figur 1; Spalte 3, Zeilen 46-63 * <br> --- | 1 | A 61 B 17/22 <br> A 61 B 8/00 |
| A | US-A-4 620 545 (W. SHENE) <br> * Figur 1; Spalte 3, Zeile 47 - Spalte 4, Zeile 13 * <br> --- | 1 | |
| A | EP-A-0 190 601 (SIEMENS AG) <br> * Zusammenfassung; Figur 1 * <br> ----- | | |

RECHERCHIERTE
SACHGEBIETE (Int. Cl.4)

A 61 B
G 01 S

Der vorliegende Recherchenbericht wurde für alle Patentansprüche erstellt

| Recherchenort | Abschlußdatum der Recherche | Prüfer |
|---|---|---|
| DEN HAAG | 20-04-1988 | NEILL M.C. |